# EUROPEAN PATENT SPECIFICATION

(11) **EP 2 000 108 B1**
(45) Date of publication and mention of the grant of the patent: **12.01.2011**
(21) Application number: 07109940.2
(22) Date of filing: 08.06.2007
(51) Int. Cl.: A61B 18/24, A61N 5/06

(54) **System for performing a method for the ablation of cartilage tissue in a knee joint using indocyanine**
System zur Ausführung eines Verfahrens zur Ablation von Knorpelgewebe am Kniegelenk unter Verwendung von Indocyanin
Système de réalisation d'un procédé pour l'ablation du tissu cartilagineux d'une articulation de genou en utilisant de l'indocyanine

(43) Date of publication of application: 10.12.2008
(73) Proprietor: Laserix Sarl, 2000 Neuchatel (CH)
(72) Inventor: Ravussin, Pierre, 1092, Belmont (CH)
(74) Representative: Ravenel, Thierry Gérard Louis

(56) References cited:
- EP-A- 1 459 694
- WO-A-98/55035
- WO-A-2007/047892
- FR-A- 2 713 836
- ZUGER B J ET AL: "Laser soldering of articular cartilage" PROCEEDINGS OF THE SPIE - THE INTERNATIONAL SOCIETY FOR OPTICAL ENGINEERING SPIE-INT. SOC. OPT. ENG USA, vol. 4244, 2001, pages 198-207, XP002456357 ISSN: 0277-786X

## Description

### Field of the Invention

The invention pertains to a system for performing a method for the ablation of cartilage tissue in a joint, such as, for example, a knee joint, of a human or animal. More particularly the invention pertains to such a system including a laser diode and an exogenous absorber applied to the surface of the cartilage tissue so as to selectively absorb electromagnetic energy from a diode laser and thereby effect ablation of cartilage tissue coated with the exogenous absorber. The invention also pertains to a new use of indocyanine green for the production of exogenous photosensitive agent intended to the ablation of human or animal cartilage tissue using laser technology

### Background of the Invention

In the orthopedic arts, it is known that cartilage tissue in a joint, such as a knee joint, has to be reshaped in order to treat certain joint disorders such as hyaline cartilage surface irregularities. Generally, arthroscopic surgery is preferred due to the lower rates of morbidity and mortality, and the shorter recovery times experienced by patients. Typically, an orthopedic surgeon will operate arthroscopically on a joint, such as the knee, by inserting one or more trocars into the joint capsule, and then through one of the trocars an arthroscope is inserted so the surgeon can visualize the tissues (i.e., synovium and cartilage) of the joint. The other trocar is used to deploy a surgical instrument, such as a mechanical cutting tool (i.e., a grasper, knife or curette). However, these mechanical cutting tools may damage a significant amount of healthy cartilage while removing damaged or diseased tissue. Because cartilage is avascular and heals slowly, there is a need to develop a tool or device that can remove damaged cartilage tissue while minimizing collateral damage to surrounding healthy cartilage.

The present inventor has considered ablation techniques involving laser technology to address this problem. For the purpose of this disclosure, "ablation" means "the act or process of ablating," and "ablating" means to remove or dissipate by vaporization or melting." The ablation of cartilage using a 308 nm Excimer laser (a UV laser) in an *in vitro* cartilage sample is disclosed by J. A. Prodoehl et al. in "308 Excimer Laser Ablation of Cartilage," Lasers Surg. Med. 15:263-268 (1994). However, Prodoehl's article does not enable one of ordinary skill in the art to perform ablation of cartilage *in vivo*. Furthermore, the Excimer laser is expensive and relatively bulky so it is not suitable for use in a hospital or outpatient surgery clinical environment. While cheaper lasers that are relatively compact and light are known, such as laser diodes for example, these lasers are not suitable for arthroscopic surgical ablation of cartilage because their wavelength energies are poorly absorbed by cartilage.

Known methods for the ablation of articular cartilage using laser technology employ crystal lasers in the infrared spectrum such as No:YAG lasers (wavelength: 2µ) and Er:YAG lasers (wavelength: 2.9 µ). However, there are several drawbacks to the application of infrared laser technology to arthroscopic surgery. First, arthroscopic surgery is performed in an aqueous environment. After entering the joint capsule with a trocar, the surgeon infuses an aqueous solution into the joint capsule so that visualization of the joint tissues with an arthroscope is optimized and to prevent excessive deterioration of the joint tissues during the procedure. Unfortunately, the aqueous liquid must be pierced by the infrared laser beam in order to reach the cartilage. In other words, the radiation of the infrared lasers must literally pierce a hole in the water by vaporizing it before reaching the cartilage to be ablated. This means that the infrared laser beam must have considerable energy, which then generates undesirable thermal effects. These undesirable thermal effects increase as the power of the laser increases, which makes quick ablation impossible without damaging a significant amount of healthy joint tissue.

A second drawback of infrared lasers relates to the difficulties encountered when selecting a laser that operates at a wavelength not absorbed well by the aqueous environment. It turns out that a laser that is not absorbed well by the aqueous environment utilized during arthroscopic surgery is also not absorbed well by articular cartilage because of the water content of articular cartilage. Therefore, there is a need to employ some means for enhancing the absorption of laser energy by damaged cartilage tissue that the surgeon wishes to ablate so that energy is preferentially absorbed by the damaged cartilage tissue and not by the healthy hyaline cartilage

The present inventor has considered the application of an exogenous absorber, such as a dye, to enhance the absorption of laser energy by cartilage tissue to effect selective tissue ablation. However, previous applications of energy absorbing dyes to therapeutic methods have been limited. For example, U.S. Patent 6,221,068 B1 to Fried et al., discloses the use of a dye such as Indocyanine Green (ICG) mixed with albumin or fibrinogen to create a tissue "solder." ICG has an absorption peak at 810 nm when combined with albumin. The tissue solder is applied to opposing edges of a wound, and a commercially available diode laser is used to activate the tissue "solder" and "weld" the tissue edges together. However, U.S. Patent 6,221,068 does not teach, or even suggest, applying the ICG/diode laser combination to effect tissue ablation. On the contrary, U.S. Patent 6,221,068 pertains to using the IGG dye to heat albumin or fibrinogen applied to wound edges to create an adhesive effect. Furthermore, U.S. Patent 6,221,068 does not teach or suggest that the tissue "solder" could be applied to a wound in an aqueous environment.

U.S. Patent Application Publication No. US 2004/0147501 to Dolmans et al. discloses generally "photodynamic therapy," which pertains to treatment methods employing a photosensitizer to generate reactive radical species to destroy cells in a target tissue. This publication discloses that ICG is a photosensitizer. The photosensitizer is injected into an injection site of a treatment subject and allowed to find its way into the subject's vasculature. After time, the photosensitizer accumulates in a highly vasularized or neovascularized target tissue, such as a rapidly growing tumor. A laser is then used to activate the photosensitizer to generate the radical species used to kill the target tissue. Photodynamic therapy, however, is not an ablation therapy. Furthermore, photodynamic therapy can only be applied to vascularized or neovascularized tissues, which most articular cartilage tissue (e.g., hyaline cartilage of the knee) is not.

WO 98/55035 discloses a method and an apparatus for ablating tissue such as cartilage using a pulsed laser beam.

The present invention endeavors to provide an improved method and system for the ablation of cartilage tissue *in vivo* in a human or animal treatment subject that overcomes the disadvantages of prior art methods and systems. Accordingly, a primary object of the present invention is to overcome the disadvantages of the prior art methods and systems for the ablation of cartilage tissue *in vivo* in a human or animal treatment subject.

Another object of the present invention is to provide a method and system for the ablation of cartilage tissue that can be employed arthroscopically in an aqueous environment within the joint capsule of a living human or animal treatment subject.

Another object of the present invention is to provide a method and system for the ablation of cartilage tissue that employs an economical and compact diode laser so that the method and system may be employed in a hospital and/or outpatient surgical environment.

Yet another object of the present invention is to provide a method and system for the ablation of cartilage that ablates cartilage tissue in a more efficient manner than achieved by prior art methods and systems.

### Summary of the Invention

In accordance with the above objectives, the present invention provides a system for the *in vivo* ablation of human or animal cartilage according to claim 1 of the patent. This systems allows the method including the steps of: (a) applying an exogenous absorber to a portion of a surface of cartilage, wherein the exogenous absorber selectively absorbs the electromagnetic energy of the laser diode at a first electromagnetic energy narrow spectrum; (b) generating electromagnetic energy at a secondary electromagnetic energy far infrared wide spectrum; and (c) directing the electromagnetic energy generated by the laser diode at the second electromagnetic energy spectrum onto the exogenous absorber applied to the portion of the surface of cartilage so that the articular cartilage absorbs the secondary electromagnetic energy causing at least partial ablation of cartilage to be performed.. The previous method embodiments can be further modified so that the cartilage is avascular articular cartilage. In accordance with yet another method embodiment, the articular cartilage of the previous embodiments is any joint cartilage.

In accordance with still another method embodiment, the previous embodiments can be further modified so that the electromagnetic energy generated by the laser diode is directed through a fluid contained within a joint capsule of a joint before being absorbed by the exogenous absorber, wherein the fluid comprises synovial fluid or an aqueous solution or a mixture of synovial fluid and the aqueous solution. In accordance with another method embodiment, the previous embodiments can be further modified so that the exogenous absorber is a dye that colors the portion of the surface of the cartilage when applied thereto, and the exogenous absorber comprises a compound having the following formula

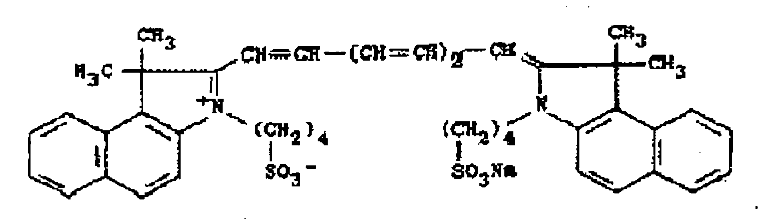

In accordance with yet another method embodiment, the previous embodiments can be further modified so that the exogenous absorber is applied to portion of the surface of articular cartilage in the form of an aqueous solution using at least one trocar inserted into a joint capsule. In accordance with still another method embodiment the previous embodiments can be modified so that the aqueous solution is a physiological fluid comprising 0.9% sodium chloride. In another method embodiment of the present invention, the aqueous solution is phosphate buffered saline.

In accordance with another method embodiment the previous embodiments can be further modified so that the exogenous absorber maximally absorbs electromagnetic energy at the first electromagnetic energy spectrum that is equal to, or approximately equal to, the second electromagnetic spectrum.

In accordance with another system embodiment of the present invention, the previous system embodiment is further modified so that the pulsed laser beam generated by the laser diode has a power of greater than 30 W. In accordance with yet another system embodiment of the present invention, the previous embodiments are further modified to include a laser device comprising the an array of laser diodes. In accordance with still another system embodiment of the present invention, the previous embodiments are further modified to include two trocars, wherein one trocar is an irrigation trocar and the other trocar is an evacuation trocar.

In accordance with another system embodiment of the present invention, the previous embodiments are further modified so that the first wavelength is around 785nm.

In accordance with another system embodiment of the present invention, the previous embodiments are further modified so that the first wavelength is at a wavelength for which a non toxic dye does exist.

The invention also proposes a new use of indocyanine green for the production of exogenous photosensitive agent intended to the ablation of human or animal cartilage tissue using laser technology. According to a particular use, the new use is characterized by the fact that the indocyanine green is applied in the ablation of human or animal cartilage tissue in a joint particularly in a knee joint.

In a particular use embodiment, the new use of indocyanine green is characterized by the fact of being applied to a portion of a surface of the cartilage.

In another use embodiment, the new use of indocyanine green is characterized by the fact is applied to portion of the surface of articular cartilage in the form of an aqueous solution.

In yet another use embodiment, the new use of indocyanine green is **characterized in that** the aqueous solution is a physiological fluid comprising 0.9% sodium chloride.

In yet another use embodiment, the new use of indocyanine green is **characterized in that** the aqueous solution is phosphate buffered saline.

In a preferred use embodiment, the new use of indocyanine green is characterized by the fact of being applied as an exogenous absorber to a portion of a surface of cartilage, wherein the exogenous absorber selectively absorbs electromagnetic energy at a first electromagnetic energy spectrum, the absorber being activated by an electromagnetic energy spectrum generated by a laser diode and causing at least partial ablation of cartilage.

Further objects, features and advantages of the present invention will become apparent from the Detailed Description of Illustrative Embodiments, which follows, when considered together with the attached drawings.

### Brief Description of the Drawings

Figure 1 is a schematic diagram of the system, in accordance with the present invention, for the ablation of cartilage tissue *in vivo* in a human or animal treatment subject.
Figure 2 illustrates the step of applying exogenous absorber to the articular cartilage of a joint.
Figure 3 illustrates the step of generating electromagnetic energy using a laser diode and directing the pulsed laser beam generated by the laser diode onto the exogenous absorber applied to a portion of the cartilage surface so that the exogenous absorber selectively absorbs electromagnetic energy causing at least partial ablation of the surface of cartilage.
Figure 4 illustrates an operating panel of a laser device that may be employed in accordance with the present invention.

### Detailed Description of the Illustrative Embodiments

The apparatus of the present invention is a system for the ablation of cartilage tissue *in vivo* in a human or animal treatment subject. While the present invention is described with respect to application to ablation of cartilage of the knee joint, the present invention is not limited in application to ablation of knee joint cartilage. The present invention may be applied to the ablation of human and/or animal cartilage in other joints as well, and even to nonarticular cartilage or to any tissue that fixes the dye.

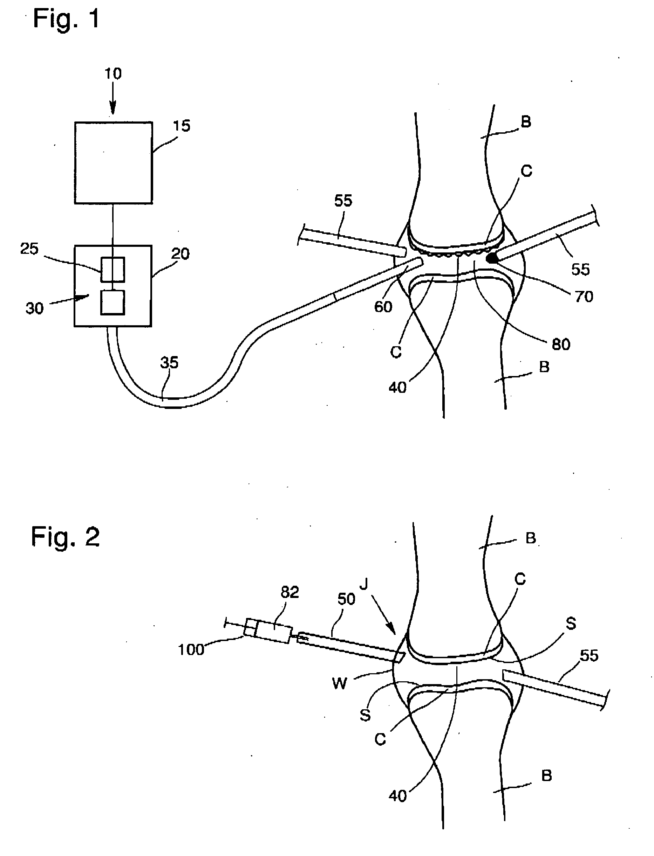

The present invention is described with reference to Figures 1 to 4 where like parts are referred to using like character references. In addition, a non-limiting illustrative apparatus embodiment, in accordance with the present invention, will be described first followed by a description of a method embodiment of using the invention.

A system 10 for ablating cartilage *in vivo* in a human or animal treatment subject includes, as shown in Figure 1, a power source 15 connected to energize a laser device 20 that includes a laser diode 25 (or an array of laser diodes 30). The laser diode 25 (or the array of laser diodes network 30) generates a pulsed laser beam having a wavelength of λ_{A}, wherein each laser pulse generated by the laser diode 25 (or the array of laser diodes 30) lasts no longer than about 10 msec and has a peak power of several hundred watts. However, the laser diode 25 (or the array of laser diodes 30) may be operated to emit a single laser pulse or a train of laser pulses. When system 10 is operated to generate a train of laser pulses (i.e., to generate a pulsed laser beam), the train of laser pulses may be adjusted in terms of duration of the train, repeat-frequency of the laser pulses, and power of the pulsed laser beam. An optical conduit 35 is connected to transmit the pulsed laser beam from the laser diode 25 (or the array of laser diodes 30) to wherever the pulsed laser beam is to be applied. Therefore, the optical conduit includes one or more flexible optical fibers for transmitting the pulsed laser beam.

The system 10 also includes an exogenous absorber that selectively absorbs electromagnetic energy at a wavelength of about λ_{B}, wherein either λ_{A} = λ_{B}, or λ_{A} ≈ λ_{B}. An exogenous absorber 40, in accordance with the present invention, is a compound or composition that includes a chromophore or dye that absorbs radiation selectively thereby confining ablation to the immediate area where the exogenous absorber is applied to cartilage tissue. In one embodiment of the present invention, the exogenous absorber includes ICG, which is a nontoxic dye already approved for medical applications and which has an absorption peak at about 785 nm when fixed to articular cartilage. ICG has a molecular formula of C₄₃H₄₇N₂NaO₆S₂ and is also known by the American Chemical Society designation CAS No. 3599-32-4. The chemical structure for ICG is:

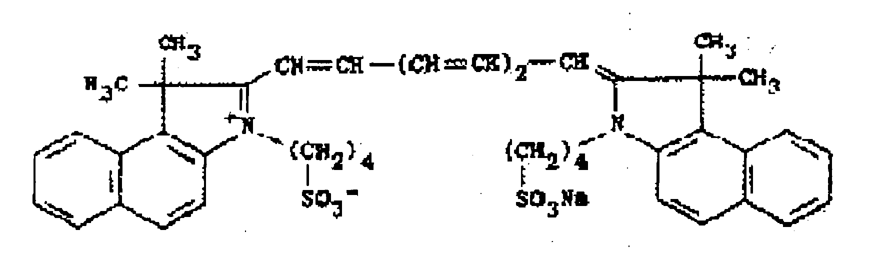

Persons of ordinary skill in the art would appreciate that other chromophores and dyes, and mixtures thereof, may be employed in the practice of the present invention so long as there is a corresponding laser diode (or array of laser diodes) that generates a pulsed laser beam that satisfies one of the relationships: λ_{A} = λ_{B}, or λ_{A} = λ_{B}.

In the case where the system 10 employs ICG, which is an exogenous absorber 40 having an absorption peak at about 785 nm, a laser diode 25 that generates a pulsed laser beam at about 785 nm is used, wherein each laser pulse generated by the laser diode lasts no more than 10 msec and has a power of up to several hundred watts. The system 10 also includes one or more trocars 50, 55 for accessing cartilage tissue to be ablated *in vivo.* When the exogenous absorber 40 is selectively applied to the synovial surface S covering cartilage tissue C on bone B, it coats or dyes the synovium S and/or the cartilage C. The ICG dye penetrates the synovial membrane S covering the cartilage C and penetrates to a depth of about 0.1 mm or less. Thus, when the pulsed laser beam, which comprises a train of laser pulses, is directed onto a portion of the cartilage C coated with the exogenous absorber 40, the exogenous absorber 40 selectively absorbs electromagnetic energy from the pulsed laser beam exiting tip 60 of optical conduit 35 thereby causing at least a portion of the irradiated and dyed cartilage to superheat to a temperature above the evaporation temperature of water. As a result, superheated cartilage vaporizes creating a vapor bubble. This vapor bubble creates tears in contiguous unvaporized cartilage causing a portion of the remaining unvaporized cartilage to disperse into a liquid medium surrounding the cartilage and contained within the joint capsule W.

The exogenous absorber ICG may be applied to the surface of cartilage C either as an aqueous solution or it may be applied directly using an applicator tool 70 deployed through one of the trocars 50, 55. As described above, the exogenous absorber ICG selectively absorbs energy from the pulsed laser beam transmitted by the optical conduit 35 and exiting tip 60 while any fluid 80 contained within the joint capsule W and any undyed cartilage absorb energy relatively poorly from the pulsed laser beam. In this way, the exogenous absorber 40 preferentially absorbs electromagnetic energy from the pulsed laser beam sufficient to achieve ablation of at least a portion of the surface of the cartilage C while undyed cartilage and the fluid 80 do not superheat.

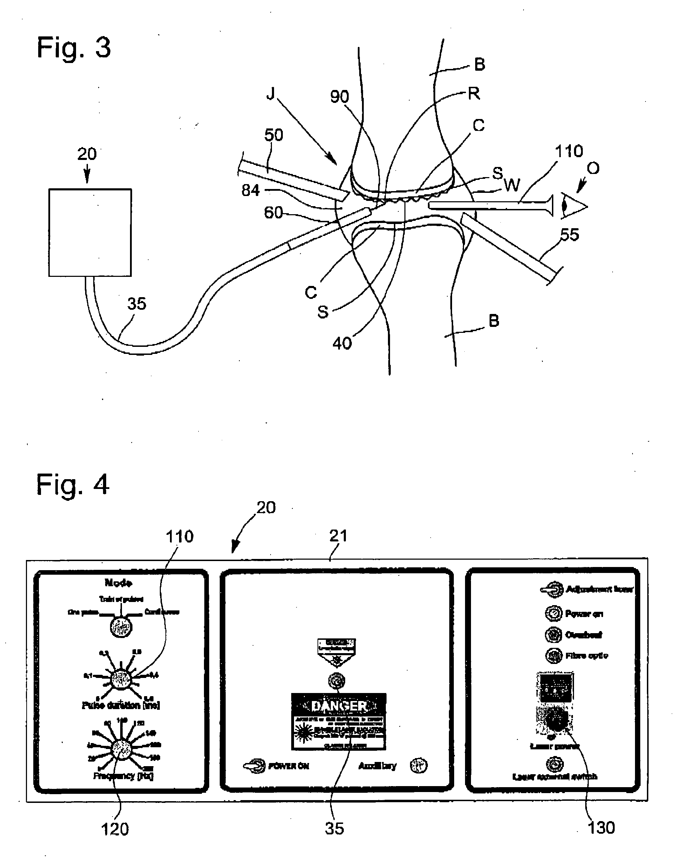

Having described the system 10, in accordance with the present invention, a method for the *in vivo* ablation of human or animal cartilage in a treatment subject will be described with reference to Figures 2 and 3 said method having been given here for explanatory purposes only. As shown in Figure 2, the method includes the steps of preparing the joint J under sterile conditions and then inserting one or more trocars 50, 55 through the joint capsule W and into the inter-articular joint space. Joint J may be a knee joint or any other joint having a joint capsule. The evacuation trocar 55 is used to evacuate synovial fluid from the joint and, within seconds, the irrigation trocar 50 is used to infuse into the inter-articular space, by means of a syringe or pump 100, a fluid 82 that comprises exogenous absorber 40 in an aqueous solution such as normal saline (0.9% NaCl) or phosphate buffered saline (PBS).

In this way, exogenous absorber is applied to all of, or a portion of, the synovial surface S of cartilage C, and then is allowed to penetrate into the cartilage C. Cartilage C is generally avascular articular cartilage, although vasularized portions of articular cartilage may be ablated using the present method as well. Typically, the exogenous absorber 40 penetrates to a depth of a few tenth of mm into the cartilage C. Alternatively, exogenous absorber may be applied directly to the synovial surface S using an applicator tool 70 deployed through the trocar 50 or 55. It is beneficial to employ an exogenous absorber 40 that is a dye that colors a portion of the surface S of the cartilage C when applied thereto so the operating surgeon may arthroscopically visually verify which portions of the articular cartilage have been dyed (i.e., have had exogenous absorber successfully applied thereto) and which portions have not been dyed.

In some cases, the exogenous absorber will preferentially dye certain defects in the cartilage C, which the surgeon O intends to ablate. This preferentially dying effect has two advantages. First, when using an exogenous absorber that is a colored dye, the dying of the defects highlights the defects and aids the surgeon O in arthroscopically identifying the defects using the arthroscope 110. Second, preferential uptake of dye by cartilage defects results in a higher concentration of dye in the defects, which enhances the accumulation of laser energy during ablation to areas containing defects.

As previously mentioned, the exogenous absorber may be any suitable compound or composition that selectively absorbs electromagnetic energy of a certain wavelength so that λ_{A} = λ_{B}, or λ_{A} ≈ λ_{B} such that the exogenous absorber absorbs sufficient energy to superheat dyed cartilage to the point of ablation whereas contiguous undyed cartilage does not absorb energy sufficient to superheat to the point of ablation. One particularly useful exogenous absorber comprises Indocyanine green (ICG), a compound having the following molecular formula, C₄₃H₄₇N₂NaO₆S₂. Suitable dyes, in addition to having a selective energy absorption spectrum, are unstable in solution so they degrade in a relatively short time. Furthermore, suitable dyes and their degradation byproducts must be nontoxic and capable of being eliminated by the treatment subject's body.

As shown in Figure 3, the method includes a step of evacuating the dye-containing fluid 82 via the evacuation trocar 55 and replacing it with a physiologic aqueous solution such as normal saline, PBS or lactated ringer's solution 84 using irrigation trocar 50. This step is carried out because the dye-containing fluid 82 is opaque to the pulsed laser beam and would selectively absorb energy and vaporize. On the other hand, the fluid 84 is used to prevent deterioration of the cartilage C during the procedure, to provide good visualization during arthroscopy, and to provide a medium through which the pulsed laser beam 90 may travel without absorbing sufficient energy from the beam 90 to vaporize. In other words, during the method of using the system of the present invention, the wavelength λ_{A} of the pulsed laser beam is chosen so that it does not readily superheat and vaporize the laser transmitting fluid 84. Laser transmitting fluid 84 is selected because it is transparent to the pulsed laser beam 90.

Once dye application fluid 82 has been replaced with laser transmitting fluid 84, the surgeon O deploys the tip 60 of the optical conduit 35 through the joint capsule W, and then energizes the laser device 20 so that the laser diode (or laser the network/array) generates electromagnetic energy (i.e., pulsed laser beam) at a first electromagnetic energy spectrum λ_{A}. The surgeon directs the pulsed laser beam 90 generated by the laser device 20 onto exogenous absorber previously applied to at least a portion of the surface S of cartilage C. The exogenous absorber, having peak absorption at λ_{B}, then selectively absorbs electromagnetic energy from the pulsed laser beam 90 thereby causing localized superheating and at least partial ablation R of cartilage C. This ablation step may be repeated on the same or other portions of the cartilage C still coated with exogenous absorber.

To further decrease the risk of collateral thermal damage to cartilage during ablation therapy, the surgeon O may irrigate the inter-articular joint space with additional fluid 84 using irrigation trocar 50 while evacuating some of the fluid 84 using evacuation trocar 55. In this way, the surgeon may flush the inter-articular joint space with fluid 84 thereby dissipating excess heat and removing debris generated by the laser ablation procedure.

Persons of ordinary skill in the art would appreciate that when an applicator tool 70 is used to apply the exogenous absorber, then use of dye application fluid 82 may be omitted altogether or applied as a pretreatment. The system of the present invention may also be used without first evacuating the synovial fluid. In such a case, the laser transmitting fluid 84 may comprise synovial fluid, or an aqueous solution, or a mixture of synovial fluid and an aqueous solution.

Persons of ordinary skill in the art would also appreciate that the duration, power, and repeat-frequency of laser pulse trains may be adjusted to suit the particular type of articular cartilage (i.e., human cartilage, horse cartilage, dog cartilage, etc.) to be ablated. For example, operating panel 21 of the laser device 20 may be provided with vernier scale switches 110, 120 as shown in Figure 4 so ablation parameters may be adjusted and optimized. For example, vernier scale switch 110 serves to adjust the duration of laser pulse trains generated by the laser diode 25 (or the array of laser diodes 30). Vernier scale switch 120 serves to adjust the repeat-frequency of the laser pulse trains. Vernier scale switch 130 serves to adjust the laser power of the pulsed laser beam and the laser pulse trains.

While the present invention has been described with reference to certain illustrative embodiments, one of ordinary skill in the art will recognize, that additions, deletions, substitutions and improvements can be made while remaining within the scope of the invention as defined by the appended claims.

## Claims

1. A system (10) for ablating cartilage in a human or animal, comprising:
a laser diode (25) adapted to generate a pulsed laser beam at about a first wavelength, wherein each laser pulse generated by the laser diode lasts no more than 10 msec;
an optical conduit (35) connected to transmit the pulsed laser beam generated by the laser diode (25); and
an exogenous absorber (40), wherein the exogenous absorber selectively absorbs electromagnetic energy at about a second wavelength so that upon application of the exogenous absorber to a surface of cartilage (C) and subjection to the pulsed laser beam transmitted by the optical conduit, the exogenous absorber absorbs electromagnetic energy from the pulsed laser beam and effects ablation of at least a portion of the cartilage, wherein the exogenous absorber (40) maximally absorbs electromagnetic energy at the wavelength of the laser diode (25) and wherein the exogenous absorber comprises indocyanine green.

2. A system according to claim 1, wherein the pulsed laser beam generated by the laser diode has a power of greater than 30 W.

3. A system according to claim 1, further comprising a laser device comprising an array of laser diodes (30).

4. A system according to claim 1, further comprising two trocars, wherein one trocar is an irrigation trocar (50) and the other trocar is an evacuation trocar (55).

5. A system according to claim 1, wherein the first wavelength and the second wavelength are each at about 785 nm.

6. A system according to claim 1, wherein the exogenous absorber comprises a non toxic dye, and the first wavelength is at a wavelength such that an electromagnetic energy absorption peak of the exogenous absorber is at the half wavelength of the laser diode.

7. A system according to claim 1, wherein the first wavelength is at a wavelength such that an electromagnetic energy absorption peak of the exogenous absorber is at the half wavelength of the laser diode.

8. Use of indocyanine green for the production of exogenous photosensitive agent intended to the ablation of human or animal cartilage tissue using laser technology.

9. Use of indocyanine green according to claim 8 **characterized by** being applicable in the ablation of human or animal cartilage tissue in a joint, particularly in a knee joint.

10. Use of indocyanine green according to claim 8 or **characterized by** being applicable to a portion of a surface of the cartilage.

11. Use of indocyanine green according to any of the preceding claims 8 to 10 **characterized by** being applicable to a portion of the surface of articular cartilage in the form of an aqueous solution.

12. Use of indocyanine green according to claim 11 **characterized in that** the aqueous solution is a physiological fluid comprising 0.9% sodium chloride.

13. Use of indocyanine green according to claim 11 **characterized in that** the aqueous solution is phosphate buffered saline.

14. Use of indocyanine green according to any of the preceding claims 8 to 13 **characterized by** being applicable as an exogenous absorber to a portion of a surface of cartilage, wherein the exogenous absorber selectively absorbs electromagnetic energy at a first electromagnetic energy spectrum, the absorber being activated by an electromagnetic energy spectrum generated by a laser diode and causing at least partial ablation of cartilage.

## Patentansprüche

1. System (10) zum Abtragen von Knorpel in einem Menschen oder Tier, das umfasst:
eine Laserdiode (25), die dazu ausgelegt ist, einen gepulsten Laserstrahl ungefähr bei einer ersten Wellenlänge zu erzeugen, wobei jeder Laserimpuls, der durch die Laserdiode erzeugt wird, nicht länger als 10 ms dauert;
eine optische Leitung (35), die so angeschlossen ist, dass sie den gepulsten Laserstrahl, der durch die Laserdiode (25) erzeugt wird, überträgt; und
einen exogenen Absorber (40), wobei der exogene Absorber elektromagnetische Energie ungefähr bei einer zweiten Wellenlänge wahlweise absorbiert, so dass bei Anwendung des exogenen Absorbers auf eine Oberfläche eines Knorpels (C) und bei Beaufschlagung mit dem durch die optische Leitung übertragenen gepulsten Laserstrahl der exogene Absorber elektromagnetische Energie aus dem gepulsten Laserstrahl absorbiert und eine Abtragung wenigstens eines Teils des Knorpels bewirkt, wobei der exogene Absorber (40) elektromagnetische Energie bei der Wellenlänge der Laserdiode (25) maximal absorbiert und wobei der exogene Absorber Indocyaningrün enthält.

2. System nach Anspruch 1, wobei der durch die Laserdiode erzeugte gepulste Laserstrahl eine Leistung von mehr als 30 W hat.

3. System nach Anspruch 1, das ferner eine Laservorrichtung enthält, die eine Anordnung von Laserdioden (30) umfasst.

4. System nach Anspruch 1, das ferner zwei Trokare enthält, wobei ein Trokar ein Irrigationstrokar (50) ist und der andere Trokar ein Evakuierungstrokar (55) ist.

5. System nach Anspruch 1, wobei die erste Wellenlänge und die zweite Wellenlänge jeweils etwa 785 nm betragen.

6. System nach Anspruch 1, wobei der exogene Absorber einen nicht giftigen Farbstoff enthält und wobei die erste Wellenlänge eine Wellenlänge ist, derart, dass eine Absorptionsspitze für elektromagnetische Energie des exogenen Absorbers bei der halben Wellenlänge der Laserdiode liegt.

7. System nach Anspruch 1, wobei die erste Wellenlänge eine Wellenlänge ist, derart, dass eine Absorptionsspitze der elektromagnetischen Energie des exogenen Absorbers bei der halben Wellenlänge der Laserdiode liegt.

8. Verwendung von Indocyaningrün für die Herstellung eines exogenen photoempfindlichen Wirkstoffs, der für die Abtragung von menschlichem oder tierischem Knorpelgewebe unter Verwendung der Lasertechnologie vorgesehen ist.

9. Verwendung von Indocyaningrün nach Anspruch 8, **dadurch gekennzeichnet, dass** sie auf die Abtragung von menschlichem oder tierischem Knorpelgewebe in einem Gelenk, insbesondere in einem Kniegelenk, anwendbar ist.

10. Verwendung von Indocyaningrün nach Anspruch 8 oder 9, **dadurch gekennzeichnet, dass** sie auf einen Abschnitt einer Oberfläche des Knorpels anwendbar ist.

11. Verwendung von Indocyaningrün nach einem der vorhergehenden Ansprüche 8 bis 10, **dadurch gekennzeichnet, dass** sie auf einen Abschnitt der Oberfläche eines Gelenkknorpels in Form einer wässrigen Lösung anwendbar ist.

12. Verwendung von Indocyaningrün nach Anspruch 11, **dadurch gekennzeichnet, dass** die wässrige Lösung ein physiologisches Fluid ist, das 0,9 % Natriumchlorid enthält.

13. Verwendung von Indocyaningrün nach Anspruch 11, **dadurch gekennzeichnet, dass** die wässrige Lösung eine mit Phosphat gepufferte Kochsalzlösung (*Saline*) ist.

14. Verwendung von Indocyaningrün nach einem der vorhergehenden Ansprüche 8 bis 13, **dadurch gekennzeichnet, dass** sie als ein exogener Absorber auf einem Abschnitt einer Oberfläche von Knorpel anwendbar ist, wobei der exogene Absorber elektromagnetische Energie in einem ersten Spektrum elektromagnetischer Energie wahlweise absorbiert, wobei der Absorber durch ein Spektrum elektromagnetischer Energie aktiviert wird, das durch eine Laserdiode erzeugt wird, und eine wenigstens teilweise Abtragung von Knorpel bewirkt.

## Revendications

1. Dispositif (10) pour l'ablation de cartilage d'un être humain ou animal, comportant :
- une diode laser (25) agencée pour générer un faisceau laser pulsé autour d'une première longueur d'onde, où chaque impulsion générée par la diode laser ne dure pas plus que 10 ms ;
- un canal optique (35) connecté pour transmettre le faisceau laser pulsé généré par la diode laser (25), et
- un absorbeur exogène (40);
**caractérisé en ce que** l'absorbeur exogène absorbe sélectivement l'énergie électromagnétique autour d'une seconde longueur d'onde, de façon à ce que, lors d'une application de l'absorbeur exogène sur la surface d'un cartilage (C), et de l'exposition au faisceau laser pulsé transmis par le canal optique, l'absorbeur exogène absorbe de l'énergie électromagnétique du faisceau laser pulsé et effectue l'ablation d'au moins une partie du cartilage,
et où l'absorbeur exogène (40) absorbe au maximum de l'énergie électromagnétique à la longueur d'onde de la diode laser (25),
et où l'absorbeur exogène comprend du vert d'indocyanine.

2. Dispositif (10) selon la revendication 1, **caractérisé en ce que** le faisceau laser pulsé généré par la diode laser a une puissance supérieure à 30 W.

3. Dispositif (10) selon la revendication 1, comportant encore un appareillage laser comportant un réseau de diodes laser (30).

4. Dispositif (10) selon la revendication 1, comportant encore deux canules, où l'une est une canule d'injection (50) et l'autre une canule d'évacuation (55).

5. Dispositif (10) selon la revendication 1, **caractérisé en ce que** la première longueur d'onde et la seconde longueur d'onde sont chacune voisines de 785 nm.

6. Dispositif (10) selon la revendication 1, **caractérisé en ce que** l'absorbeur exogène comporte une teinture non toxique, et **en ce que** la première longueur d'onde est telle qu'un pic d'absorbtion d'énergie électromagnétique de l'absorbeur exogène est à la demi-longueur d'onde de la diode laser.

7. Dispositif (10) selon la revendication 1, **caractérisé en ce que** la première longueur d'onde est telle qu'un pic d'absorbtion d'énergie électromagnétique de l'absorbeur exogène est à la demi-longueur d'onde de la diode laser.

8. Utilisation du vert d'indocyanine pour la production d'un agent photosensible exogène destiné à l'ablation d'un tissu cartilagineux humain ou animal en utilisant la technologie laser.

9. Utilisation du vert d'indocyanine selon la revendication 8, **caractérisée en ce qu'**elle est applicable à l'ablation d'un tissu cartilagineux humain ou animal dans une jointure, en particulier à la jointure du genou.

10. Utilisation du vert d'indocyanine selon la revendication 8 ou 9, **caractérisée en ce qu'**elle est applicable à une portion de la surface du cartilage.

11. Utilisation du vert d'indocyanine selon l'une quelconque des revendications 8 à 10, **caractérisée en ce qu'**elle est applicable à une portion de surface de cartilage articulaire sous la forme d'une solution aqueuse.

12. Utilisation du vert d'indocyanine selon la revendication 11, **caractérisée en ce que** la solution aqueuse est un fluide physiologique comprenant 0.9% de chlorure de sodium.

13. Utilisation du vert d'indocyanine selon la revendication 11, **caractérisée en ce que** la solution aqueuse est une solution saline tamponnée en phosphate.

14. Utilisation du vert d'indocyanine selon l'une quelconque des revendications 8 à 13, **caractérisée en ce qu'**elle est applicable en tant qu'absorbeur exogène à une portion de surface de cartilage, et **en ce que** l'absorbeur exogène absorbe sélectivement de l'énergie électromagnétique à un premier spectre d'énergie électromagnétique, l'absorbeur étant activé par un spectre d'énergie électromagnétique généré par une diode laser et occasionnant l'ablation au moins partielle du cartilage.
